# EUROPÄISCHE PATENTSCHRIFT

(11) **EP 2 089 530 B1**
(45) Veröffentlichungstag und Bekanntmachung des Hinweises auf die Patenterteilung: **03.03.2010**
(21) Anmeldenummer: 07822458.1
(22) Anmeldetag: 09.11.2007
(51) Int. Cl.: C12P 23/00, C12N 9/04

(54) **VERFAHREN ZUR HERSTELLUNG VON (4S)-3,4-DIHYDROXY-2,6,6-TRIMETHYL-CYCLOHEX-2-ENON UND DERIVATEN DAVON UNTER VERWENDUNG DER AZOARCUS PHENYLETHANOL DEHYDROGENASE**
PROCESS FOR THE PREPARATION OF (4S)-3,4-DIHYDROXY-2,6,6-TRIMETHYL-CYCLOHEX-2-ENONE AND DERIVATIVES THEREOF EMPLOYING THE AZOARCUS PHENYLETHANOL DEHYDROGENASE
PROCÉDÉ DE PRÉPARATION DE (4S)-3,4-DIHYDROXY-2,6,6-TRIMÉTHYL-CYCLOHEX-2-ÉNONE ET DE SES DÉRIVÉS EN UTILISANT LA PHÉNYLÉTHANOL DÉSHYDROGÉNASE DE AZOARCUS

(30) Priorität: 10.11.2006 EP 06123814
(43) Veröffentlichungstag der Anmeldung: 19.08.2009
(73) Patentinhaber: BASF SE, 67056 Ludwigshafen (DE)
(72) Erfinder: BREUER, Michael, 64285 Darmstadt (DE); ERNST, Hansgeorg, 67346 Speyer (DE); HAUER, Bernhard, 67136 Fussgönheim (DE)
(86) Internationale Anmeldenummer: PCT/EP2007/062170
(87) Internationale Veröffentlichungsnummer: WO 2008/055988

(56) Entgegenhaltungen:
- WO-A-2005/108590
- ZELL, R. ET AL.: "Technische Verfahren zur Synthese von Carotinoiden und verwandten Verbindungen aus 6-Oxo-isophoron. III. Ein neues Konzept für die Synthese der enantiomeren Astaxanthine // Technical Procedures for the Synthesis of Carotenoids and Related Compounds for 6-oxo-isophorones" HELVETICA CHIMICA ACTA, Bd. 64, Nr. 7, 1. Januar 1981 (1981-01-01), Seiten 2447-2462, XP001202861 in der Anmeldung erwähnt

## Beschreibung

Die vorliegende Erfindung betrifft ein Verfahren zur Herstellung von optisch aktiven (4S)-4-Hydroxy-2,6,6-trimethyl-cyclohex-2-en-1-on-Derivaten der Formel (I) und ein Verfahren zur Herstellung von (3S, 3'S)-Astaxanthin der Formel (III) umfassend das erstgenannte Verfahren.

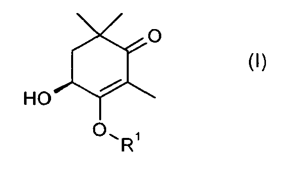

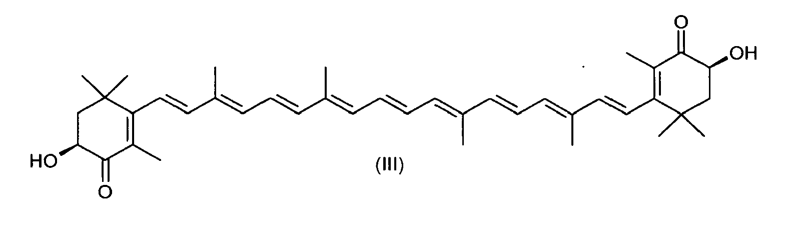

Aufgrund seiner zwei Chiralitätszentren in 3 und 3'-Position kann Astaxanthin (3,3'-Dihydroxy-β,β'-carotin-4,4'-dion) in Form folgender Konfigurationsisomere vorliegen: (3S,3'S), (3R,3'R), (3S,3'R) und (3R,3'S). Die beiden letztgenannten Konfigurationsisomere sind identisch und stellen eine meso-Form dar (Carotenoids Handbook, 2004, Main List Nr. 405).

Alle drei Formen werden in natürlichen Quellen gefunden (Carotenoids Handbook, 2004, Main List Nr. 404, 405, 406). Die chemische Totalsynthese, ausgehend von racemischen Vorprodukten, führt zu einem 1:2:1-Gemisch aus (3S,3'S)-, meso- und (3R,3'R)-Astaxanthin (The EFSA Journal, 2005, 291, 12, Deutsche Lebensmittel-Rundschau, 2004, 100, 437, Helvetica Chimica Acta, 1981, 64, 2436).

Von besonderer Bedeutung ist jedoch das (3S,3'S)-Konfigurationsisomer. Es wird von Grünalgen (Haematococcus pluvialis) in enantiomerenreiner Form biosynthetisiert (J. Applied Phycology, 1992, 4, 165; Phytochemistry, 1981, 20, 2561).

(S,S)-Astaxanthin aus Grünalgen wird als Nahrungsmittelergänzungsstoff mit positiven Effekten für die menschliche Gesundheit eingesetzt (J. Nat. Prod., 2006, 69, 443). Darüber hinaus ist es geeignet, die nachteiligen prooxidativen Effekte von Rofecoxib (Vioxx) vollständig zu blockieren (J. Cardiovasc. Pharmacol., 2006, 47 Suppl 1, S. 7).

Angesichts der geringen Konzentration von (S,S)-Astaxanthin in Grünalgen (J. Agric. Food Chem., 1998, 46, 3371) ist die Verfügbarkeit dieses Wirkstoffes jedoch sehr begrenzt. Zudem liegt der Wirkstoff in den Algen in einem Gemisch aus Mono- und Di-Fettsäureestern sowie freiem Astaxanthin vor, was einen erheblichen Aufwand zur Isolierung und Aufreinigung verursacht (s.u.a. Phytochemistry, 20, 11, 2561 (1981); J.Applied Phycology, 4, 2, 165 (1992)). Um (S,S)-Astaxanthin in größerer Menge und hoher Reinheit zur Verfügung stellen zu können, ist die chemische Totalsynthese die Technologie der Wahl.

Verschiedene Synthesen von (S,S)-Astaxanthin wurden in der Literatur beschrieben. Eine Strategie besteht in der Spaltung racemischer Vorstufen in die optischen Antipoden, wobei diastereomere Salze (Helvetica Chimica Acta, 1981, 64, 2447) oder diastereomere Ester (Helvetica Chimica Acta, 1981, 64, 2419) verwendet wurden. Auch über die mikriobielle Racematspaltung racemischer Vorstufen wurde berichtet. Besonders nachteilig an diesen Verfahren ist, dass das Enantiomer, das zu (R,R)-Astaxanthin führen würde, nicht brauchbar ist bzw. nur sehr aufwendig rückgeführt werden kann.

Eine andere Synthesestrategie besteht darin, über mikrobielle bzw. enzymatische Verfahren zu enantiomerenreinen Synthesebausteinen zu gelangen (Helvetica Chimica Acta, 1978, 61, 2609, Helvetica Chimica Acta, 1981, 64, 2405) Da diese Bausteine einen zu niedrigen Oxidationszustand aufweisen, mussten sie in vielstufigen Synthesen zu (S,S)-Astaxanthin-Vorprodukten umgewandelt werden.

WO 2006/039685 beschreibt zum einen in Schema II eine zweistufige enantioselektive Hydrierung von Ketoisophoron zu einem enantiomerenreinen C9-Diol, aus dem man nach Re-Oxidation einer Hydroxygruppe in Anlehnung an das in Helv.Chim.Acta,1978 61, 2609 beschriebene Verfahren in einer vielstufigen Synthese zu (S,S)-Astaxanthin-Vorstufen gelangt. Weiterhin beschreibt WO 2006/039685 eine enantioselektive katalytische Transferhydrierung eines C9-Enolethers der Formel (IIa) zum entsprechenden enantiomerenreinen Alkohol der Formel (la).

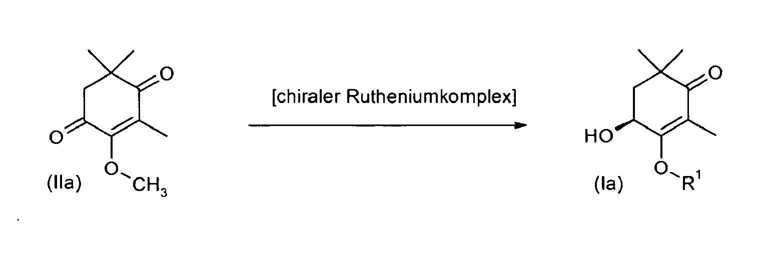

Als Hydrierkatalysatoren werden Metalle mit chiralen Liganden, vorzugsweise Ruthenium-Katalysatoren mit optisch aktiven Aminen als Liganden, beschrieben. Nachteilig an diesem Verfahren ist, dass ein O-geschütztes Derivat eines technischen Zwischenproduktes der Formel (IIb) eingesetzt wird, 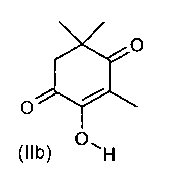 was zusätzlichen synthetischen Aufwand erfordert. Zudem sind die benötigten optisch aktiven Katalysatoren sehr teuer, so dass ihre Verwendung in technischen Verfahren unter ökonomischen Gesichtspunkten erschwert wird.

Auch die biokatalytische Reduktion des Cg-Enols der Formel (IIb) mit Hefe wurde beschrieben. Dabei wurde jedoch nur ein Enantiomerenüberschuss von 65% erhalten, was diesen Prozess für ein technisches Verfahren unbrauchbar macht. (Helv.Chim.Acta, 1981, 64, 2447).

Prinzipiell ist bekannt, dass sich Dehydrogenasen als Biokatalysatoren zur Herstellung optisch aktiver Hydroxyverbindungen eignen. Es handelt sich um gut charakterisierte Biokatalysatoren, die bereits in einer Reihe von technischen Prozessen eingesetzt werden (Angew. Chem. Int. Ed., 2004, 43, 788, Tetrahedron, 2004, 60, 633, Chiral catalysis - asymmetric hydrogenation supplement to Chemistry Today, 2004, 22, 26, Current Opinion in Chemical Biology, 2004, 8, 120, Organic Process Research & Development, 2002, 6, 558, Tetrahedron: Asymmetry, 2003, 14, 2659, Chiral catalysisasymmetric hydrogenation supplement to Chemistry Today, 2004, 22, 43).

In WO 2005/108590 wird ein Verfahren zur Herstellung bestimmter optisch aktiver Alkanole, wie beispielsweise (1S)-3-Methylamino-1-(2-thienyl)-propan-1-ol oder (1S)-3-Chlor-1-(2-thienyl)-propan-1-ol, durch enzymatische Reduktion der entsprechenden Ketone beschrieben. Inwieweit die verwendeten Enzyme zur Reduktion strukturell andersartiger Ketone verwendbar sind, wird nicht diskutiert.

Die beschriebenen Verfahren zur Einführung der beiden Chiralitätszentren in eine der Vorstufen für die Synthese von S,S'-Astaxanthin sind entweder komplex und vielstufig oder unwirtschaftlich, so dass diese Verfahren für eine Realisierung im technischen Maßstab wenig geeignet erscheinen.

Es war Aufgabe der vorliegenden Erfindung, ausgehend von technisch verfügbaren Ausgangsprodukten ein einfaches und ökonomisch effizientes Verfahren zur Herstellung eines optisch aktiven Zwischenproduktes, möglichst in enantiomerenreiner Form, für die Synthese von (S,S')-Astaxanthin zu entwickeln, das sich problemlos in die existierenden technischen Totalsynthesen von "racemischem" Astaxanthin (Carotenoids Vol.2, 1996, 259; Pure and Applied Chemistry, 2002, 74, 2213) integrieren lässt.

Diese Aufgabe wird durch ein Verfahren zur Herstellung von optisch aktiven (4S)-4-Hydroxy-2,6,6-trimethyl-cyclohex-2-en-1-on-Derivaten der Formel (I) 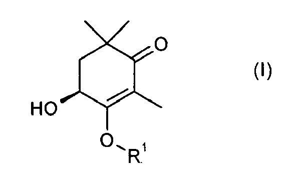 worin
R¹ für Wasserstoff, C₁-C₁₀-Alkyl, C₇-C₁₄-Arylalkyl, ein Alkalimetall M¹ oder ein Erdalkalimetallfragment M²_{1/2} oder (M²)⁺X⁻ steht, wobei M¹ für Li, Na, K, Rb oder Cs und M² für Mg, Ca, Sr oder Ba steht und X- ein einfach geladenes Anion bedeutet,
umfassend einen Reaktionsschritt,
wobei man in einem Medium, das ein Trimethyl-cyclohex-2-en-1,4-dion-Derivat der Formel (II) enthält, 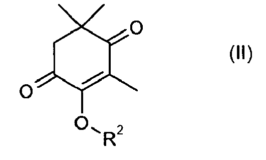 worin R² gleich oder verschieden von R¹ ist und für Wasserstoff, C₁-C₁₀-Alkyl, C₇-C₁₄-Arylalkyl, ein Alkalimetall M¹ oder ein Erdalkalimetallfragment M²_{1/2} oder (M²)⁺X- steht, wobei M¹ für Li, Na, K, Rb oder Cs und M² für Mg, Ca, Sr oder Ba steht und X- ein einfach geladenes Anion bedeutet,
ein Enzym (E) in Gegenwart von Reduktionsäquivalenten inkubiert, wobei die Verbindung der Formel (II) zur Verbindung der Formel (I) enzymatisch reduziert wird, und die im Laufe der Reaktion verbrauchten Reduktionsäquivalente durch Umsetzen eines Reduktionsmittels (RM) zum entsprechenden Oxidationsprodukt (OP) mit Hilfe des Enzyms (E) oder eines weiteren Enzyms (E²) wieder regeneriert werden und optional das Oxidationsprodukt (OP) zumindest partiell aus dem Reaktionsmedium oder aus dem Reaktionsgleichgewicht entfernt wird, und man das gebildete Produkt (I) isoliert,
wobei das Enzym (E) eine Polypeptidsequenz besitzt, die entweder
(i) SEQ ID NO: 2 ist, oder
(ii) bei der bis zu 25% der Aminosäurereste gegenüber SEQ ID NO: 2 durch Deletion, Insertion, Substitution oder eines Kombination davon verändert sind, und die noch mindestens 50% des enzymatischen Aktivität von SEQ ID NO: 2 aufweist,
gelöst.

In dem erfindungsgemäßen Verfahren werden Verbindungen der Formel (I) hergestellt, worin R¹ für Wasserstoff, C₁-C₁₀-Alkyl, wie beispielsweise Methyl, Ethyl, n-Propyl, iso-Propyl oder n-Hexyl, C₇-C₁₄-Arylalkyl, wie beispielsweise Benzyl, ein Alkalimetall M¹ oder ein Erdalkalimetallfragment M²_{1/2} oder (M²)⁺X- steht, wobei M¹ für Li, Na, K, Rb oder Cs, bevorzugt Na oder K, insbesondere Na und M² für Mg, Ca, Sr oder Ba, insbesondere Mg steht und X- ein einfach geladenes Anion, wie beispielsweise Halogenid, Acetat oder Dihydrogenphosphat bedeutet. Bevorzugt steht R¹ für Wasserstoff, Methyl, Na oder K, besonders bevorzugt für Wasserstoff, Methyl oder Na, insbesondere für Wasserstoff oder Natrium.

In den Ausgangsverbindungen der Formel (II), die in dem erfindungsgemäßen Verfahren umgesetzt werden, ist der Rest R² gleich oder verschieden von R¹ und steht für Wasserstoff, C₁-C₁₀-Alkyl, wie beispielsweise Methyl, Ethyl, n-Propyl, iso-Propyl oder n-Hexyl, C₇-C₁₄-Arylalkyl, wie beispielsweise Benzyl, ein Alkalimetall M¹ oder ein Erdalkalimetallfragment M²_{1/2} oder (M²)⁺X-, wobei M¹ für Li, Na, K, Rb oder Cs, bevorzugt Na oder K, insbesondere Na und M² für Mg, Ca, Sr oder Ba, insbesondere Mg steht und X-ein einfach geladenes Anion, wie beispielsweise Halogenid, Acetat oder Dihydrogenphosphat bedeutet. Bevorzugt steht R¹ für Wasserstoff, Methyl, Na oder K, besonders bevorzugt für Wasserstoff, Methyl oder Na, insbesondere für Wasserstoff oder Natrium.

Weitere geeignete Enzyme (E) der Klasse der Oxidoreduktasen, das heißt insbesondere Enzyme mit Dehydrogenase-Aktivität, sind insbesondere die Enzyme der Familien der Aldo-Keto-Reduktasen der Aldo-Keto-Reduktase-Superfamily (K.M.Bohren, B.Bullock, B.Wermuth und K.H.Gabbay J.Biol. Chem. 1989, 264, 9547-9551) und der kurzkettigen Alkoholdehydrogenasen / Reduktasen (shortchain alcohol dehydrogenases / reductases [SDR]). Die letztere Enzymgruppe ist zum Beispiel ausführlich beschrieben bei H.Jörnvall, B.Persson, M.Krook, S.Atrian, R.Gonzalez-Duarte, J.Jeffery und D.Ghosh, Biochemistry, 1995, 34, S. 6003-6013 oder U.Oppermann, C.Filling, M.Hult, N.Shafqat, X.Q.Wu, M.Lindh, J.Shafqat, E.Nordling, Y.Kallberg, B.Persson und H.Jornvall, Chemico-Biological Interactions, 2003, 143, S. 247-253. Innerhalb dieser genannten Enzymklassen sind die Alkoholdehydrogenasen, insbesondere die kurzkettigen Alkoholdehydrogenasen besonders gut geeignet. Unter den Alkoholdehydrogenasen sind insbesondere die Enzyme geeignet, die mit NADH oder NADPH als Reduktionsäquivalente die Verbindung der Formel (II) zur Verbindung der Formel (I) reduzieren.

Geeignete Enzyme (E) mit Oxidoreduktase-Aktivität, insbesondere Dehydrogenase-Aktivität, die eine Aminosäuresequenz gemäß SEQ ID NO: 2 umfassen, sowie "funktionale Äquivalente" oder Analoga der konkret offenbarten Enzyme (E) mit Oxidoreduktase-Aktivität, insbesondere Dehydrogenase-Aktivität, die ebenfalls in dem Verfahren eingesetzt werden können, werden ausführlich in WO 2005/108590, Seite 11 bis 16 beschrieben, worauf hier ausdrücklich Bezug genommen wird.

Vorzugsweise verwendet man in dem erfindungsgemäßen Verfahren ein Enzym (E), wobei das Enzym (E) eine Polypeptidsequenz besitzt, die entweder
(i) SEQ ID NO: 2 ist, oder
(ii) bei der bis zu 25% der Aminosäurereste gegenüber SEQ ID NO: 2 durch Deletion, Insertion, Substitution oder eines Kombination davon verändert sind, und die noch mindestens 50% des enzymatischen Aktivität von SEQ ID NO: 2 aufweist,
das aus Mikroorganismen der Gattungen Azoarcus, Azonexus, Azospira, Azovibrio, Dechloromonas, Ferribacterium, Petrobacter, Propionivibrio, Quadricoccus, Rhodocyclus, Sterolibacterium, Thauera und Zoogloea herstellbar ist.

Besonders bevorzugt wird in dem erfindungsgemäßen Verfahren ein Enzym (E), wobei das Enzym (E) eine Polypeptidsequenz besitzt, die entweder
(i) SEQ ID NO: 2 ist, oder
(ii) bei der bis zu 25% der Aminosäurereste gegenüber SEQ ID NO: 2 durch Deletion, Insertion, Substitution oder eines Kombination davon verändert sind, und die noch mindestens 50% des enzymatischen Aktivität von SEQ ID NO: 2 aufweist,
welches ausgewählt ist unter Enzymen aus Mikroorganismen der Gattung Azoarcus, insbesondere aus dem Bakterium Azoarcus sp.EbN1.

Aufgrund ihrer Aminosäuresequenz kann man die Phenylethanol-Dehydrogenase aus Azoarcus sp EbN1 zu den kurzkettigen Alkoholdehydrogenasen / Reduktasen (shortchain alcohol dehydrogenases / reductases [SDR]) zählen. Die Enzymgruppe ist zum Beispiel ausführlich beschrieben bei H.Jörnvall, B.Persson, M.Krook, S.Atrian, R.Gonzalez-Duarte, J.Jeffery und D.Ghosh, Biochemistry, 1995, 34, S. 6003-6013 oder U.Oppermann, C.Filling, M.Hult, N.Shafqat, X.Q.Wu, M.Lindh, J.Shafqat, E.Nordling, Y.Kallberg, B.Persson und H.Jornvall, Chemico-Biological Interactions, 2003, 143, S. 247-253. Innerhalb der Gruppe der SDR können sich die Aminosäuresequenzen der einzelnen Vertreter stark voneinander unterscheiden. Dennoch ist bekannt, dass bestimmte Aminosäuren oder Aminosäurenbereiche innerhalb der Gruppe der SDR stark konserviert sind. In C.Filling, K.D.Berndt, J.Benach, S.Knapp, T.Prozorovski, E.Nordling, R.Ladenstein, H.Jornvall und U.Oppermann, Journal of Biological Chemistry, 2002, 277, S. 25677-25684 sind wichtige konservierte Bereiche der SDR beschrieben.

Beispiele für Azoarcus-Arten sind Azoarcus anaerobius, Azoarcus buckelii, Azoarcus communis, Azoarcus evansii, Azoarcus indigens, Azoarcus toluclasticus, Azoarcus tolulyticus, Azoarcus toluvorans, Azoarcus sp., Azoarcus sp. 22Lin, Azoarcus sp. BH72, Azoarcus sp. CC-11, Azoarcus sp. CIB, Azoarcus sp. CR23, Azoarcus sp. EB1, Azoarcus sp. EbN1, Azoarcus sp. FL05, Azoarcus sp. HA, Azoarcus sp. HxN1, Azoarcus sp. mXyN1, Azoarcus sp. PbN 1, Azoarcus sp. PH002, Azoarcus sp. T und Azoarcus sp. ToN1.

Besonders bevorzugt verwendet man in dem erfindungsgemäßen Verfahren als Enzym (E) Dehydrogenasen aus dem Bakterium Azoarcus sp.EbN1, wobei das Enzym (E) eine Polypeptidsequenz besitzt, die entweder
(i) SEQ ID NO: 2 ist, oder
(ii) bei der bis zu 25% der Aminosäurereste gegenüber SEQ ID NO: 2 durch Deletion, Insertion, Substitution oder eines Kombination davon verändert sind, und die noch mindestens 50% des enzymatischen Aktivität von SEQ ID NO: 2 aufweist.

Das erfindungsgemäße Verfahren wird bevorzugt in Gegenwart eines Enzyms (E) durchgeführt, wobei das Enzym von einer Nukleinsäuresequenz gemäß SEQ ID NO:1 oder einem funktionalen Äquivalent davon kodiert wird.

Die Nukleinsäuresequenzen, die für die Kodierung der in dem Verfahren verwendbaren Enzyme (E) mit Dehydrogenase-Aktivität eingesetzt werden können, werden ausführlich in WO 2005/108590, Seite 16 bis 22 beschrieben, worauf hier ausdrücklich Bezug genommen wird.

In einer besonders bevorzugten Ausführungsform des Verfahrens ist das Enzym mit Dehydrogenase-Aktivität ausgewählt unter Enzymen, welche eine Aminosäuresequenz gemäß SEQ ID NO: 2 umfassen oder eine davon abgeleitete Sequenz, bei der bis zu 25%, bevorzugt bis zu 20%, besonders bevorzugt bis zu 15% insbesondere bis zu 10, 9, 8, 7, 6, 5, 4, 3, 2,1 % der Aminosäurereste durch eine Deletion, eine Substitution eine Insertion oder eine Kombination aus Deletion, Substitution und Insertion verändert worden sind, wobei die gegenüber SEQ ID NO: 2 veränderten Polypeptidsequenzen noch mindestens 50%, bevorzugt 65%, besonders bevorzugt 80% , insbesondere mehr als 90 % der enzymatischen Aktivität von SEQ ID NO:2 besitzten. In diesem Zusammenhang soll unter enzymatischer Aktivität von SEQ ID NO:2 die Fähigkeit verstanden werden, die Ketone der Formel (II), insbesondere mit R¹ = H oder Na enantioselektiv zu dem (S)-Alkohol mit der allgemeinen Formel (I) zu reduzieren.

Das erfindungsgemäße Verfahren wird unter Zugabe von Reduktionsäquivalenten, insbesondere von NADH oder NADPH, die als Hydridquelle dienen, durchgeführt. Da es sich bei NADH oder NADPH um sehr teure Verbindungen handelt, werden diese Reduktionsäquivalente üblicherweise nur in katalytischen Mengen eingesetzt. Zur Regenerierung der bei der Reaktion verbrauchten Reduktionsäquivalente können als Reduktionsmittel (RM) prinzipiell anorganische oder organische Verbindungen, wie beispielsweise Phosphite oder Alkohole, oder auch elektrochemische Verfahren, wie die Reduktion an einer Kathode, eingesetzt werden. Bevorzugt wird in dem erfindungsgemäßen Verfahren als Reduktionsmittel (RM) eine organische Verbindung, die mindestens eine primäre oder sekundäre Alkoholfunktion CH(OH) enthält, wie beispielsweise Isopropanol, 2-Butanol, 2-Pentanol, 2-Hexanol, 3-Hexanol oder reduzierende Zucker wie Glucose, insbesondere Isopropanol oder Glucose, eingesetzt. Das Reduktionsmittel (RM) wird mit Hilfe des Enzyms (E) oder eines weiteren Enzyms (E²) zum Oxidationsprodukt (OP) umgesetzt, wobei das Oxidationsprodukt (OP) zumindest partiell aus dem Reaktionsmedium oder aus dem Reaktionsgleichgewicht entfernt wird. Handelt es sich bei dem Reduktionsmittel (RM) um einen sekundären Alkohol, so bezeichnet man diesen auch häufig als Opfer-Alkohol und das entsprechend gebildete Oxidationsprodukt (OP) als Opfer-Keton.

In einer bevorzugten Ausführungsform der Erfindung wird der zugesetzte Opfer-Alkohol nicht nur zur Regenerierung der verbrauchten Reduktionsäquivalente eingesetzt, sondern auch als Co-Lösungsmittel. Man kann in einem flüssigen 1-Phasen-, 2-Phasen- oder auch Mehrphasensystem arbeiten, wobei üblicherweise eine Phase aus Wasser und/oder einem mit Wasser mischbaren Lösungsmittel besteht.

Die Reduktionsäquivalente werden bevorzugt in einer Menge von 0,001 bis 100 mmol, besonders bevorzugt von 0,01 bis 1 mmol Reduktionsäquivalente pro Mol eingesetztem Trimethyl-cyclohex-2-en-1,4-dion-Derivat der Formel (II) eingesetzt.

Bei dem erfindungsgemäßen Verfahren kann das gebildete Oxidationsprodukt (OP) zumindest partiell aus dem Reaktionsmedium oder aus dem Reaktionsgleichgewicht entfernt werden. Im Falle von sekundären Alkoholen wie Isopropanol als Reduktionsmittel (RM) kann die Entfernung der sogenannten, gebildete Opfer-Ketone, Aceton im Falle von Isopropanol als Opferalkohol, auf verschiedene Weise vorgenommen werden, beispielsweise durch selektive Membranen oder durch Extraktions- oder Destillationsverfahren. Bevorzugt wird die Destillation zur Entfernung eines Ketons wie Aceton eingesetzt. Üblicherweise wird bei der destillativen Abtrennung des Ketons auch ein Teil des Opfer-Alkohols und zum Teil auch noch Teile der wässrigen Phase mit entfernt. Diese Destillationsverluste werden in der Regel durch Nachdosieren des Opfer-Alkohols und ggf. des Wassers ausgeglichen. Die Destillationsraten liegen üblicherweise in einem Bereich von 0,02%/min bis 2%/min, bevorzugt von 0,05%/min bis 1 %/min bezogen auf das Reaktionsvolumen. Die Manteltemperaturen des Reaktors liegen zwischen 5 - 70 Kelvin, bevorzugt zwischen 10 - 40 Kelvin über der Reaktorinnentemperatur.

Im Falle eines nicht flüchtigen Oxidationsproduktes (OP), wie im Falle der Oxidation von Glucose zur Gluconsäure wird letztere durch Cyclisierung zum Gluconolacton dem Reaktionsgleichgewicht zwischen Reduktionsmittel (RM) und Oxidationsmittel (OM) entzogen. Während die Oxidation vieler Opferalkohole durch dasselbe Enzym (E) möglich ist, das auch die Reduktion von Verbindungen der Formel (II) zu Verbindungen der Formel (I) katalysiert, muss für die Oxidation von Glucose ein zweites Enzym (E²) wie beispielsweise Glucosedehydrogenase zugeführt werden.

Im Falle von flüchtigen Oxidationsprodukten wird die Destillation besonders gut in einem Druckbereich von 1-500 mbar, bevorzugt 10-200 mbar durchgeführt.

Eine bevorzugte Ausführungsform des erfindungsgemäßen Verfahrens ist es, dass die Umsetzung der Verbindung der Formel (II) zur Verbindung der Formel (I), in Gegenwart eines Mikroorganismus erfolgt, der ausgewählt ist unter Bakterien der Familien Enterobacteriaceae, Pseudomonadaceae, Rhizobiaceae, Lactobacillaceae, Streptomycetaceae, Rhodococcaceae, Rhodocyclaceae und Nocardiaceae. Der Mikroorganismus kann insbesondere ein rekombinanter Mikroorganismus sein, der mit einem Nukleinsäurekonstrukt transformiert ist, welches für ein Enzym kodiert, wobei das Enzym (E) eine Polypeptidsequenz besitzt, die entweder
(i) SEQ ID NO: 2 ist, oder
(ii) bei der bis zu 25% der Aminosäurereste gegenüber SEQ ID NO: 2 durch Deletion, Insertion, Substitution oder eines Kombination davon verändert sind, und die noch mindestens 50% des enzymatischen Aktivität von SEQ ID NO: 2 aufweist.

Expressionskonstrukte, die unter der genetischen Kontrolle regulativer Nukleinsäuresequenzen eine Nukleinsäuresequenz enthalten, die für ein in dem Verfahren verwendbaren Protein kodiert, d. h. für ein Enzym (E), sowie entsprechende Vektoren, die wenigsten eines der Expressionskonstrukte umfassen, werden ausführlich in WO 2005/108590, Seite 22 bis 25 beschrieben, worauf hier ausdrücklich Bezug genommen wird.

Rekombinante Mikroorganismen, die mit einem geeigneten Vektor oder Konstrukt transformiert sind und die zur Produktion der in dem Verfahren verwendbaren Polypeptide, d. h eines Enzyms (E), eingesetzt werden können, werden ausführlich in WO 2005/108590, Seite 25 bis 27 beschrieben, worauf hier ausdrücklich Bezug genommen wird.

Verfahren zur rekombinanten Herstellung von Polypeptiden oder funktioneller, biologisch aktiver Fragmente davon, die in dem Verfahren die Funktion des Enzyms (E) erfüllen, wobei man einen Polypeptide-produzierenden Mikroorganismus kultiviert, gegebenenfalls die Expression der Polypeptide induziert und diese aus der Kultur isoliert, werden ausführlich in WO 2005/108590, Seite 27 bis 29 beschrieben, worauf hier ausdrücklich Bezug genommen wird. Die Polypeptide können nach den angegebenen Methoden auch in großtechnischem Maßstab produziert werden.

Die erfindungsgemäß verwendeten Enzyme (E), wobei das Enzym (E) eine Polypeptidsequenz besitzt, die entweder
(i) SEQ ID NO: 2 ist, oder
(ii) bei der bis zu 25% der Aminosäurereste gegenüber SEQ ID NO: 2 durch Deletion, Insertion, Substitution oder eines Kombination davon verändert sind, und die noch mindestens 50% des enzymatischen Aktivität von SEQ ID NO: 2 aufweist,
können im erfindungsgemäßen Verfahren als freies oder immobilisiertes Enzym (E) verwendet werden.

Eine bevorzugte Ausführungsform des erfindungsgemäßen Verfahren umfasst wenigsten die Schritte a), b) und d) der nachfolgend genannten Schritte:
a) einen ein Enzym wobei das Enzym (E) eine Polypeptidsequenz besitzt, die entweder
   (i) SEQ ID NO: 2 ist, oder
   (ii) bei der bis zu 25% der Aminosäurereste gegenüber SEQ ID NO: 2 durch Deletion, Insertion, Substitution oder eines Kombination davon verändert sind, und die noch mindestens 50% des enzymatischen Aktivität von SEQ ID NO: 2 aufweist,
   produzierenden Mikroorganismus aus einer natürlichen Quelle zu isolieren oder rekombinant herzustellen,
b) diesen Mikroorganismus zu vermehren,
c) aus dem Mikroorganismus das Enzym gegebenenfalls zu isolieren oder eine dieses Enzym enthaltende Proteinfraktion herzustellen, und
d) den Mikroorganismus gemäß Stufe b) oder das Enzym gemäß Stufe c) in ein Medium zu überführen, das eine Verbindung der Formel I enthält.

Das erfindungsgemäße Verfahren wird vorteilhaft bei einer Temperatur zwischen 0°C und 95°C, bevorzugt zwischen 10°C und 85°C, besonders bevorzugt zwischen 15°C und 75°C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten bzw. optisch aktiven Alkoholen sind im erfindungsgemäßen Verfahren Enantiomere zu verstehen, die eine Enantiomerenanreicherung zeigen. Im erfindungsgemäßen Verfahren werden bevorzugt Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die geeignete Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme (E) können für das Verfahren verwendet werden. Ebenfalls geeignet sind immobilisierte Mikroorganismen oder Enzyme, die vorteilhaft in der Reaktion Anwendung finden können.

Werden für das erfindungsgemäße Verfahren freie Organismen oder Enzyme verwendet, so werden diese vor der Extraktion zweckmäßigerweise abgetrennt, beispielsweise über eine Filtration oder Zentrifugation.

Die in dem erfindungsgemäßen Verfahren hergestellten Verbindungen der Formel (I), beispielsweise (3S)-3,4-Dihydroxy-2,6,6-trimethyl-cyclohex-2-enon, lassen sich vorteilhaft aus der wässrigen Reaktionslösung über Extraktion oder Fällung gewinnen. Vorteilhaft wird die Produktlösung zur Abtrennung nicht gelösten biologischen Materials zunächst filtriert, vorzugsweise unter Zusatz eines Filterhilfsmittels wie Celite.
Die Abtrennung des Produkts (mit R₁ = Alkyl) erfolgt anschließend durch Extraktion mit einem mit Wasser nicht mischbaren organischen Lösungsmittel. Beispiele für geeignete Lösungsmittel sind Toluol oder andere cyclische oder offenkettige Kohlenwasserstoffe, chlorierte Kohlenwasserstoffe wie beispielsweise Methylenchlorid, Ethyl- oder Butylacetat, sowie Ether wie MTBE oder Diisopropylether.

Die Isolierung der Verbindung der Formel I (mit R¹ = H oder Alkali- oder ErdalkaliMetall) aus der Reaktionslösung kann prinzipiell so erfolgen wie in Helv.Chim.Acta 64, 2436, 1981 beschrieben. Die Produktlösung wird zunächst auf einen pH von 1 bis 3, vorzugsweise pH 1, gestellt. Das Ansäuern wird vorzugsweise mit Mineralsäuren wie etwa Salz- oder Schwefelsäure, besonders bevorzugt mit Schwefelsäure, durchgeführt. Dabei fällt das Produkt aus und kann abgetrennt werden.
Bevorzugt wird die saure Produktlösung aber mehrmals mit einem organischen Lösungsmittel extrahiert. Als Lösungsmittel sind hier chlorierte Kohlenwasserstoffe, insbesondere Methylenchlorid , Ether wie etwa MTBE oder Disiiopropylether sowie Essigester geeignet. Diese Extraktion kann diskontinuierlich oder kontinuierlich durchgeführt werden. Die Extraktion des Produkts kann durch Aufkonzentrierung der wässrigen Phase vor dem Ansäuern oder durch "Aussalzen" unterstützt werden; diese Operationen sind aber nicht essentiell für die Abtrennung des Produkts aus der Reaktionslösung.

Bei den genannten Aufarbeitungsverfahren lässt sich das Produkt der Formel (I) des erfindungsgemäßen Verfahrens in Ausbeuten von 60 bis >95%, bevorzugt von 80 bis 95%, bezogen auf das für die Reaktion eingesetzte Substrat der Formel (II) (wie z.B. mit R² = Na), isolieren. Das Produkt hat eine hohe Enantiomerenreinheit von > 98% ee. Es kann gewünschenfalls durch Kristallisation gemäß Helv. Chim. Acta 64, 2436, 1981 aufgereinigt werden, wird aber vorzugsweise ohne weitere Reinigungsoperation in der weiteren Synthese von S,S-Astaxanthin gemäß Helv. Chim. Acta 64, 2447, 1981 eingesetzt.

Das erfindungsgemäße Verfahren kann batchweise, semi-batchweise oder kontinuierlich betrieben werden.

Die Durchführung des erfindungsgemäßen Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Ein weiterer Gegenstand der vorliegenden Erfindung ist ein Verfahren zur Herstellung von (3S, 3'S)-Astaxanthin der Formel (III) umfassend das oben beschriebene Verfahren zur Herstellung von optisch aktiven (4S)-4-Hydroxy-2,6,6-trimethyl-cyclohex-2-en-1-on-Derivaten der Formel (I) als einem Reaktionsschritt der Gesamtsynthese von (3S, 3'S)-Astaxanthin. Sowohl die Syntheseschritte zur Herstellung der Ausgangsverbindungen der Formel (II) als auch die Syntheseschritte zur Umsetzung der enantiomeren-reinen Verbindung der Formel (I) über mehrere Stufen zu (3S, 3'S)-Astaxanthin der Formel (III) sind prinzipiell literaturbekannt. Die Überführung der durch enantioselektive Reduktion von (II), worin R² vorzugsweise Na ist, erhaltenen optisch reinen Verbindung der Formel (I), worin R¹ gleich Wasserstoff ist, in (3S,3'S)-Astaxanthin erfolgt racemisierungsfrei wie in der Literatur verschiedentlich beschrieben (WO 2006/039685; Helv.Chim.Acta, 1981, 64, 2447; ibid., 1981, 64, 2405).
Diese Verfahren entsprechen technischen Astaxanthin-Synthesen (Carotenoids, Vol.2, 1996, 259; Pure and Appl.Chem., 2002, 74, 2213) und bieten einen technisch wie ökonomisch vorteilhaften Zugang zu (3S,3'S)-Astaxanthin.

Die vorliegende Erfindung betrifft auch die Verwendung eines Enzyms (E) mit einer Polypeptidsequenz, die entweder
(i) SEQ ID NO: 2 ist, oder
(ii) bei der bis zu 25% der Aminosäurereste gegenüber SEQ ID NO:2 durch Deletion, Insertion, Substitution oder einer Kombination davon verändert sind, und die noch mindestens 50% der enzymatischen Aktivität von SEQ ID NO:2 aufweist,
zur Herstellung von Verbindungen der Formel (I). Bevorzugt wird die Verwendung des oben beschriebenen Enzyms/zur Herstellung von Verbindungen der Formel (I) in einem Verfahren zur Herstellung von (3S, 3'S)-Astaxanthin, wobei die Verbindung der Formel (I) in weiteren Reaktionsschritten zu (3S, 3'S)-Astaxanthin der Formel (III) umgesetzt wird.

Der Vorteil des erfindungsgemäßen Verfahrens liegt in der Gewinnung von Verbindungen der Formel (I) mit hoher Enantiomerenreinheit verbunden mit guten Ausbeuten an diesen Verbindungen.

Die Erfindung wird durch folgende Beispiele erläutert.

### Beispiele

### Definitionen:

Verbindung 1: 2-Hydroxy-3,5,5-trimethyl-cyclohex-2-en-1,4-dion (Formel (II) mit R² gleich Wasserstoff)
Verbindung 1a: Natrium-3,5,5-trimethyl-1,4-dioxo-cyclohex-2-en-2-olat (Formel (II) mit R² gleich Natrium)
Verbindung 1b: 2-Methoxy-3,5,5-trimethylcyclohex-2-en-1,4-dion (Formel (II) mit R² gleich Methyl)
Verbindung 2: (4S)-3,4-Dihydroxy-2,6,6-trimethyl-cyclohex-2-enon (Formel (I) mit R¹ gleich Wasserstoff)
Verbindung 2b: (4S)-4-Hydroxy-3-methoxy-2,6,6-trimethyl-cyclohex-2-enon (Formel (I) mit R¹ gleich Methyl)

### Beispiel 1: Bereitstellung rekombinanter Phenylethanol-Dehydrogenase

E. coli LU11558, hergestellt wie in WO 2005/108590, Beispiel 1 und 2 beschrieben, wurde in 20mL LB-Amp/Spec/Cm (100µg/l Ampicillin; 100µg/l Specti-nomycin; 20µg/l Chloramphenicol), 0,1mM IPTG, 0,5g/L Rhamnose in 100mL Erlen-meyerkolben (Schikanen) 18 h bei 37°C angezogen, bei 5000*g/10min zentrifugiert, einmal mit 10mM TRIS*HCl, pH 7,0 gewaschen und in 2 mL des gleichen Puffers re-suspendiert. Zellfreier Proteinrohextrakt wurde hergestellt, indem Zellpaste von E. coli LU11558 mit 0,7ml Glaskugeln (d=0,5mm) in einer Schwingmühle (3x 5min mit Zwischenkühlung auf Eis) aufgeschlossen wurde.

### Beispiel 2: Aktivitätsbestimmung der rekombinanten Dehydrogenase aus E. coli LU11558

10 µL zellfreier Rohextrakt (Beispiel 1; ca. 10mg/mL Gesamtprotein) wurden 480 min in einer Mischung aus 770 µl 50mM K-Phosphat-Puffer (mit 1 mM MgCl₂, pH6,5), 100µL i-Propanol, 100 µl NADH-Lösung (0,5M) und 20µL Verbindung 1 (1 M in DMSO) schüttelnd inkubiert. Die Ansätze wurden analog zu Beispiel 3 analysiert. Im Durchschnitt wurden 0,13 mM 3,4-Dihydroxy-2,6,6-trimethyl-cyclohex-2-enon gebildet. In Kontrollversuchen ohne Zusatz von Rhamnose bei der Anzucht war kein Umsatz detektierbar.

### Beispiel 3 Analytik der Verbindungen 1 und 2

Die Edukt- und Produktkonzentration lassen sich mittels HPLC bestimmen. Je nach Wahl der stationären und mobilen Phase lassen sich neben der Konzentration auch ee-Wert bestimmen.

| | | | | | |
|---|---|---|---|---|---|
| stationäre Phase: | Chiralpak AS-RH, 150*4,6mm, Daicel, temperiert auf 40°C | | | | |
| mobilePhase: | Laufmittel A: 10mM KH₂PO₄ | | | | |
| | Laufmittel B: CH₃CN | | | | |

| | Gradient: | Zeit [min] | A[%] | B[%] | Fluß [ml/min] |
|---|---|---|---|---|---|
| | | 0 | 90 | 10 | 0,5 |
| | | 10 | 90 | 10 | 0,5 |
| | | 11 | 60 | 40 | 0,5 |
| | | 20 | 60 | 40 | 0,5 |
| | | | | | |
| Flussrate: | 0,5 ml/min | | | | |
| Detektion: | UV-Detektion bei 260nm | | | | |
| Retentionszeiten: | | | | | |
| | (+)-3,4-Dihydroxy-2,6,6-trimethyl-cyclohex-2-enon: | | | | ca. 9,3 min |
| | (-)-3,4-Dihydroxy-2,6,6-trimethyl-cyclohex-2-enon: | | | | ca. 9,8 min |
| | 2-Hydroxy-3,5,5-trimethyl-cyclohex-2-en-1,4-dion: | | | | ca. 17,6 min |

Mit authentischem Material wird eine Eichreihe erstellt, anhand derer die Konzentration unbekannter Proben bestimmt werden kann und die Zuordnung der Enantiomere ermöglicht wird.

### Beispiel 4: Bereitstellung von Glucose-Dehydrogenase zur Cofaktor-Regenerierung

Zur Kofaktor-Regenerierung kann Glucose-Dehydrogenase verwendet werden. Das Enzym ist aus kommerziellen (z.B. Jülich Fine Chemicals Order-No. 22.10 oder 19.10) oder eigenen Quellen zugänglich. Bei letzterem handelt es sich um ein E. coli XL10 Gold Klon der im Plasmid pUC19 das Glucose-Dehydrogenase-Gen aus Bacillus subtilis (Genbank-Acc.No. M12276) enthält (dieses Konstrukt trägt die Bezeichnung E. coli LU11293).

Zur Fermentation von E. coli LU11293 wurde folgendes Medium angesetzt:

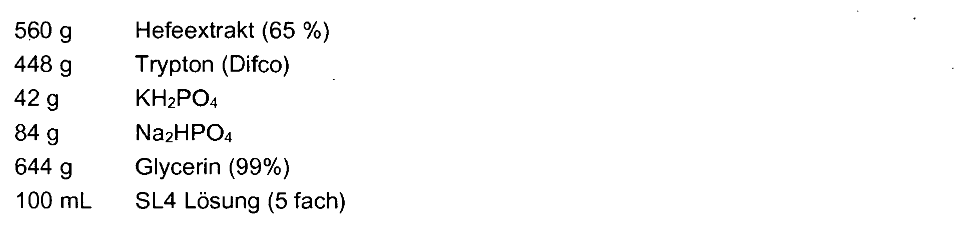

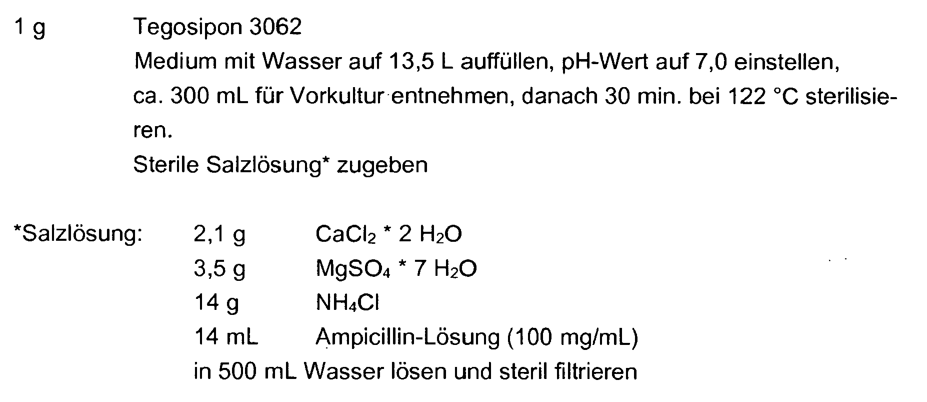

Jeweils 150 mL Medium wurden in zwei 1 L Erlenmeyerkolben sterilisiert und mit 5 mL steriler Salzlösung komplettiert. Nach Beimpfen von einer LB-Ampicillin-Agarplatte wurden die Vorkulturen 12 Stunden bei 37 °C und 200 UPM inkubiert und zum Fermentationsmedium gegeben. Die Fermentation wurde bei 37°C, 0,1 bar Innendruck, pH 7,0 (Regelung mit 20% Phosphorsäure und 25% NaOH) mit einer Begasungsrate von 7,5 L/min und 300 upm gestartet (Regelung pO₂ zwischen 20 und 50% mit 10-20 L/min Zuluft und 500-1500 Upm). Nach 2 h wurden zur Induktion 0,1 mM IPTG zugegeben und nach insgesamt 13 h die Fermentation beendet. Nach Ernte und Waschen der Zellen (1,3 kg) wurden diese bis zur Verwendung (2-20 g/L im Ansatz) bei -20°C gelagert.

### Beispiel 5: Kofaktorregenerierung

Die Regenerierung des Kofaktors kann auch durch die Phenylethanol-Dehydrogenase durchgeführt werden. In diesem Fall ist die Zugabe eines gesonderten Regenerationsenzyms nicht notwendig. Die Phenylethanol-Dehydrogenase akzeptiert verschiedene einfache Alkohole als Reduktionsmittel. Sie werden zu den entsprechenden Carbonylverbindungen oxidiert. Ein einfacher Alkohol der sich zur Regeneration von NADH mit Phenylethanol-Dehydrogenase eignet ist iso-Propanol. Wird anstelle von Glucosedehydrogenase und Glucose 10% iso-Propanol im Reaktionsansatz verwendet, kann die Aktivität der Phenylethanol-Dehydrogenase wie in Beispiel 2 gezeigt bestimmt werden.

### Beispiel 6: Herstellung von (4S)-3,4-Dihydroxy-2,6,6-trimethyl-cyclohex-2-enon-(Verbindung 2) mit der rekombinanten Phenylethanol-Dehydrogenase aus Azoarcus sp EbN1

E. coli LU11558 wurde wie in Beispiel 1 angezogen, geerntet und zu zellfreiem Rohextrakt umgearbeitet. Dieser wurde mit 0,2 mM NAD+ und 5,4 mL einer 1,68 M Natrium-3,5,5-trimethyl-1,4-dioxo-cyclohex-2-en-2-olat-Lösung (Verbindung 1a) versetzt und 48 h bei 30°C inkubiert.
Zellfreier Rohextrakt von LU11558 (≈ 250 mg Gesamtprotein)

| | |
|---|---|
| 19,8 g | Glukose |
| 0,072 g | NAD, Na-Salz |

GDH Rohextrakt ( ≈ 180 mg Gesamtprotein)

| | |
|---|---|
| 450mL | Pufferlösung (50mM K-Phosphat, 1 mM MgCl₂ pH 6,5) |
| 5,4 mL | Natrium-2-Hydroxy-3,5,5-trimethyl-4-oxo-cyclohex-2-enolat-Lösung (1,68M) |

Nach 4.75 h wurden 5,4 mL bzw. nach 6 h 16,2 mL der Substratlösung zugegeben. Die Reaktion wurde durch Titration mit 5 M NaOH und 1 M HCI bei pH 6,0-7,0 gehalten und durch HPLC-Analytik verfolgt.

### Beispiel 7: Herstellung von (4S)-4-Hydroxy-3-methoxy-2,6,6-trimethyl-cyclohex-2-enon mit einer rekombinanten Dehydrogenase aus Azoarcus sp EbN1

E. coli LU11558 wurde wie in Beispiel 1 angezogen, geerntet und zu zellfreiem Rohextrakt umgearbeitet. Dieser wurde mit 0,2 mM NAD+ und 5,4 mL einer 1,68M Natrium-3,5,5-trimethyl-1,4-dioxo-cyclohex-2-en-2-olat-Lösung versetzt und 48 h bei 30°C inkubiert.
Zellfreier Rohextrakt von E. coli LU11558 (≈ 20 mg Gesamtprotein)

| | |
|---|---|
| 19,8 g | Glukose |
| 0,014 g | NAD, Na-Salz |
| 10 mL | i-Propanol |
| 450mL | Pufferlösung (50mM K-Phosphat, 1mM MgCl₂ pH 6,5) |
| 1,822 g | 2-Methoxy-3,5,5-trimethylcyclohex-2-en-1,4-dion (Verbindung 1 b) |

Die Reaktion wurde durch HPLC-Analytik verfolgt. Nach 7 Stunden wurde die Eduktmenge durch Zugabe von 3,6 g 2-Methoxy-3,5,5-trimethylcyclohex-2-en-1,4-dion aufgestockt. Nach 75 Stunden war das Edukt zu mehr als 60% verbraucht.

### Beschreibung der Figuren:

Fig I stellt SEQ ID NO:1 dar, was die Nukleinsäuresequenz der Phenylethanol-Dehydrogenase aus Azoarcus sp EbN1 (Genbank ID 25956124, Region:25073 bis 25822) ist.
Fig II stellt SEQ ID NO:2 dar, die die Aminosäuresequenz der Phenylethanol-Dehydrogenase aus Azoarcus sp EbN1 (Genbank protein ID CAD58337) ist.

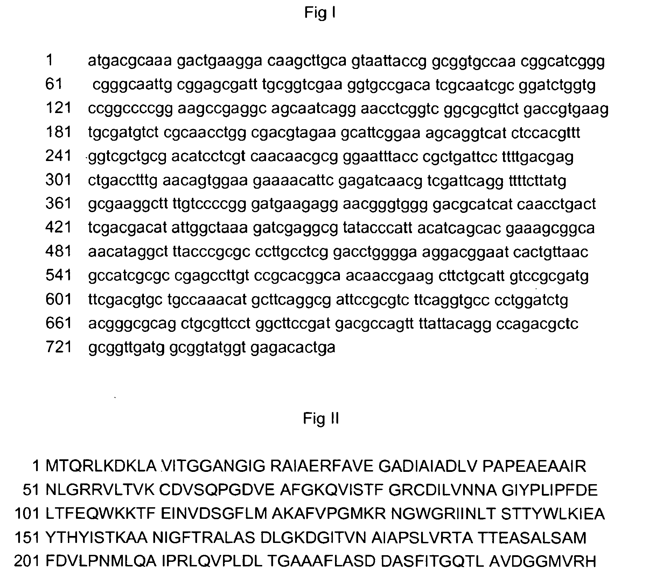

## Patentansprüche

1. Verfahren zur Herstellung von optisch aktiven (4S)-4-Hydroxy-2,6,6-trimethylcyclohex-2-en-1-on-Derivaten der Formel (I) 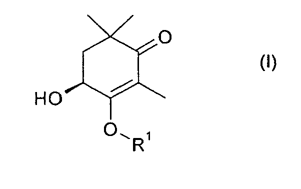 worin
R¹ für Wasserstoff, C₁-C₁₀-Alkyl, C₇-C₁₄-Arylalkyl, ein Alkalimetall M¹ oder ein Erdalkalimetallfragment M²_{1/2} oder (M²)⁺X- steht, wobei M¹ für Li, Na, K, Rb oder Cs und M² für Mg, Ca, Sr oder Ba steht und X- ein einfach geladenes Anion bedeutet,
umfassend einen Reaktionsschritt,
wobei man in einem Medium, das ein Trimethyl-cyclohex-2-en-1,4-dion-Derivat der Formel (II) enthält, 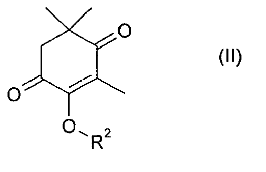 worin R² gleich oder verschieden von R¹ ist und für Wasserstoff, C₁-C₁₀-Alkyl, C₇-C₁₄-Arylalkyl, ein Alkalimetall M¹ oder ein Erdalkalimetallfragment M²_{1/2} oder (M²)⁺X- steht, wobei M¹ für Li, Na, K, Rb oder Cs und M² für Mg, Ca, Sr oder Ba steht und X- ein einfach geladenes Anion bedeutet,
ein Enzym (E), das eine Polypeptidsequenz besitzt, die entweder
(i) SEQ ID NO: 2 ist, oder
(ii) bei der bis zu 25% der Aminosäurereste gegenüber SEQ ID NO:2 durch Deletion, Insertion, Substitution oder einer Kombination davon verändert sind, und die noch mindestens 50% der enzymatischen Aktivität von SEQ ID NO:2 aufweist,
in Gegenwart von Reduktionsäquivalenten inkubiert, wobei die Verbindung der Formel (II) zur Verbindung der Formel (I) enzymatisch reduziert wird, und die im Laufe der Reaktion verbrauchten Reduktionsäquivalente durch Umsetzen eines Reduktionsmittels (RM) zum entsprechenden Oxidationsprodukt (OP) mit Hilfe des Enzyms (E) oder eines weiteren Enzyms (E²) wieder regeneriert werden und optional das Oxidationsprodukt (OP) zumindest partiell aus dem Reaktionsmedium oder aus dem Reaktionsgleichgewicht entfernt wird, und man das gebildete Produkt (I) isoliert.

2. Verfahren nach Anspruch 1, wobei das Enzym von einer Nukleinsäuresequenz gemäß SEQ ID NO:1 oder einem funktionalen Äquivalent davon kodiert wird.

3. Verfahren nach einem der vorhergehenden Ansprüche, wobei als Reduktionsmittel (RM) eine organische Verbindung, die mindestens eine primäre oder sekundäre Alkoholfunktion CH(OH) enthält, insbesondere Isopropanol oder Glucose, eingesetzt wird.

4. Verfahren nach einem der vorhergehenden Ansprüche, wobei die Umsetzung der Verbindung der Formel (II) in Gegenwart eines Mikroorganismus erfolgt, der ausgewählt ist unter Bakterien der Familien Enterobacteriaceae, Pseudomonadaceae, Rhizobiaceae, Lactobacillaceae, Streptomycetaceae, Rhodococcaceae, Rhodocyclaceae und Nocardiaceae.

5. Verfahren nach einem der vorhergehenden Ansprüche, wobei der Mikroorganismus ein rekombinanter Mikroorganismus ist, der mit einem Nukleinsäurekonstrukt transformiert ist, welches für ein Enzym gemäß der Definition in Anspruch 1 kodiert.

6. Verfahren nach einem der vorhergehenden Ansprüche, wobei R¹ in Formel (I) für Wasserstoff, Methyl oder Natrium, insbesondere Wasserstoff oder Natrium steht.

7. Verfahren zur Herstellung von (3S, 3'S)-Astaxanthin umfassend ein Verfahren nach einem der vorhergehenden Ansprüche als einem Reaktionsschritt der Gesamtsynthese von (3S, 3'S)-Astaxanthin.

8. Verwendung eines Enzyms (E) mit einer Polypeptidsequenz
i. SEQ ID NO: 2 oder
ii. bei der bis zu 25% der Aminosäurereste gegenüber SEQ ID NO:2 durch Deletion, Insertion, Substitution oder einer Kombination davon verändert sind und die noch mindestens 50% der enzymatischen Aktivität von SEQ ID NO:2 aufweist,
zur Herstellung einer Verbindung der Formel (I).

9. Verwendung nach Anspruch 8, wobei die Verbindung der Formel (I) in weiteren Reaktionsschritten zu (3S, 3'S)-Astaxanthin der Formel (III) umgesetzt wird

## Claims

1. A method for preparing optically active {4S}-4-hydroxy-2,6,6-trimethylcyclohex-2-en-1-one derivatives of the formula (I) 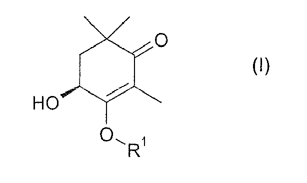 in which
R¹ is hydrogen, C₁-C₁₀-alkyl, C₇-C₁₄-arylalkyl, an alkali metal M¹ or an alkaline earth metal fragment M²_{½} or (M²)⁺X⁻, where M¹ is Li, Na, K, Rb or Cs and M² is Mg, Ca, Sr or Ba, and X⁻ is a singly charged anion,
comprising a reaction step
where an enzyme (E) which has a polypeptide sequence which either
(i) is SEQ ID NO: 2, or
(ii) in which up to 25% of the amino acid residues are altered by comparison with SEQ ID NO:2 by deletion, insertion, substitution or a combination thereof and which still has at least 50% of the enzymatic activity of SEQ ID NO:2, is incubated in a medium which comprises a trimethylcyclohex-2-ene-1,4-dione derivative of the formula (II) 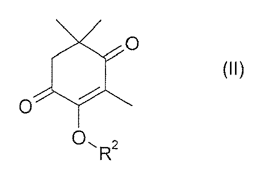 in which R² is identical to or different from R¹ and is hydrogen, C₁-C₁₀-alkyl, C₇-C₁₄-arylalkyl, an alkali metal M¹ or an alkaline earth metal fragment M²_{½}or (M²)⁺X,⁻ where M¹ is Li, Na, K, Rb or Cs and M² is Mg, Ca, Sr or Ba, and X⁻ is a singly charged anion,
in the presence of reducing equivalents, with the compound of the formula (II) being enzymatically reduced to the compound of the formula (I), and the reducing equivalents consumed during the course of the reaction are regenerated again by converting a reducing means (RM) into the corresponding oxidation product (OP) with the aid of the enzyme (E) or of a further enzyme (E²), and optionally the oxidation product (OP) is at least partially removed from the reaction medium or from the reaction equilibrium, and the product (I) which is formed is isolated.

2. The method according to claim 1, where the enzyme is encoded by a nucleic acid sequence as shown in SEQ ID NO:1 or a functional equivalent thereof.

3. The method according to any of the preceding claims, where an organic compound which comprises at least one primary or secondary alcohol function CH(OH), in particular isopropanol or glucose, is employed as reducing means (RM).

4. The method according to any of the preceding claims, where the conversion of the compound of the formula (II) takes place in the presence of a microorganism which is selected from bacteria of the families Enterobacteriaceae, Pseudomonadaceae, Rhizobiaceae, Lactobacillaceae, Streptomycetaceae, Rhodococcaceae, Rhodocyclaceae and Nocardiaceae.

5. The method according to any of the preceding claims, where the microorganism is a recombinant microorganism which is transformed with a nucleic acid construct which codes for an enzyme as defined in claim 1.

6. The method according to any of the preceding claims, where R¹ in formula (I) is hydrogen, methyl or sodium, especially hydrogen or sodium.

7. A method for preparing (3S, 3'S)-astaxanthin comprising a method according to any of the preceding claims as one reaction step in the overall synthesis of (3S,3'S)-astaxanthin.

8. The use of an enzyme (E) having a polypeptide sequence
i. SEQ ID NO: 2 or
ii. in which up to 25% of the amino acid residues are altered by comparison with SEQ ID NO:2 by deletion, insertion, substitution or a combination thereof and which still has at least 50% of the enzymatic activity of SEQ ID NO:2,
for preparing a compound of the formula (I).

9. The use according to claim 8, where the compound of the formula (I) is converted in further reaction steps into (35,3'S)-astaxanthin of the formula (III).

## Revendications

1. Procédé pour la préparation de dérivés optiquement actifs de la (4S)-4-hydroxy-2,6,6-triméthylcyclohex-2-én-1-one de formule (I) 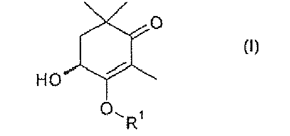 où
R¹ représente hydrogène, C₁-C₁₀-alkyle, C₇-C₁₄-arylalkyle, un métal alcalin M¹ ou un fragment de métal alcalino-terreux M²_{1/2} ou (M²)⁺X⁻, où M¹ représente Li, Na, K, Rb ou Cs et M² représente Mg, Ca, Sr ou Ba et X⁻ signifie un anion monochargé,
comprenant une étape de réaction,
dans laquelle on incube dans un milieu qui contient un dérivé de triméthylcyclohex-2-ène-1,4-dione de formule (II), 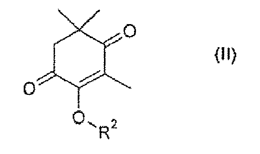 dans laquelle R² est identique ou différent de R¹ et représente hydrogène, C₁-C₁₀-alkyle, C₇-C₁₄-arylalkyle, un métal alcalin M¹ ou un fragment de métal alcalino-terreux M²_{1/2} ou (M²)⁺X⁻, où M¹ représente Li, Na, K, Rb ou Cs et M² représente Mg, Ca, Sr ou Ba et X⁻ signifie un anion monochargé,
une enzyme (E), qui présente une séquence polypeptidique
(i) qui présente la séquence SEQ ID NO : 2, ou
(ii) dans laquelle jusqu'à 25% des restes d'acides aminés sont modifiés par rapport à la séquence SEQ ID NO :2 par délétion, insertion, substitution ou une combinaison de celles-ci et qui présente encore au moins 50% de l'activité enzymatique de la SEQ ID NO :2,
en présence d'équivalents de réduction, où le composé de formule (II) est réduit enzymatiquement en composé de formule (I) et les équivalents de réduction consommés au cours de la réaction sont à nouveau régénérés par transformation d'un agent de réduction (RM) en produit d'oxydation correspondant (OP) à l'aide de l'enzyme (E) ou d'une autre enzyme (E²) et le produit d'oxydation (OP) est éventuellement éliminé, du moins partiellement, du milieu de réaction ou de l'équilibre de réaction, et on isole le produit (I) formé.

2. Procédé selon la revendication 1, l'enzyme étant codée par une séquence d'acides nucléiques selon la séquence SEQ ID NO :1 ou un équivalent fonctionnel de celle-ci.

3. Procédé selon l'une quelconque des revendications précédentes, où on utilise comme agent de réduction (RM) un composé organique qui contient au moins une fonction alcool primaire ou secondaire CH(OH), en particulier l'isopropanol ou le glucose.

4. Procédé selon l'une quelconque des revendications précédentes, où la transformation du composé de formule (II) est réalisée en présence d'un microorganisme, qui est choisi parmi les bactéries des familles des Enterobacteriaceae, des Pseudomonadaceae, des Rhizobiaceae, des Lactobacillaceae, des Streptomycetaceae, des Rhodococcaceae, des Rhodocyclaceae et des Nocardiaceae.

5. Procédé selon l'une quelconque des revendications précédentes, où le microorganisme est un microorganisme recombinant, qui est transformé avec un élément d'acide nucléique qui code pour une enzyme selon la définition dans la revendication 1.

6. Procédé selon l'une quelconque des revendications précédentes, où R¹ dans la formule (I) représente hydrogène, méthyle ou sodium, en particulier hydrogène ou sodium.

7. Procédé pour la préparation de (3S,3'S)-astaxanthine comprenant un procédé selon l'une quelconque des revendications comme une étape de réaction de la synthèse totale de la (3S,3'S)-astaxanthine.

8. Utilisation d'une enzyme (E) présentant une séquence polypeptidique
i. qui présente la séquence SEQ ID NO : 2, ou
ii. dans laquelle jusqu'à 25% des restes d'acides aminés sont modifiés par rapport à la séquence SEQ ID NO :2 par délétion, insertion, substitution ou une combinaison de celles-ci et qui présente encore au moins 50% de l'activité enzymatique de la SEQ ID NO :2,
pour la préparation d'un composé de formule (I).

9. Utilisation selon la revendication 8, où le composé de formule (I) est transformé dans d'autres étapes de réaction en (3S, 3'S)-astaxanthine de formule (III).
